(19) Europäisches Patentamt
European Patent Office
Office européen des brevets

(11) EP 4 245 328 A1

(12) EUROPEAN PATENT APPLICATION
published in accordance with Art. 153(4) EPC

(43) Date of publication:
**20.09.2023 Bulletin 2023/38**

(21) Application number: **22736901.4**

(22) Date of filing: **07.01.2022**

(51) International Patent Classification (IPC):
***A61L 27/52*** (2006.01) ***A61L 27/20*** (2006.01)
***C08B 37/08*** (2006.01) ***C08J 3/075*** (2006.01)
***A61M 5/28*** (2006.01)

(52) Cooperative Patent Classification (CPC):
**A61L 27/20; A61L 27/52; A61M 5/28; C08B 37/003; C08J 3/075**

(86) International application number:
**PCT/KR2022/000338**

(87) International publication number:
**WO 2022/149924 (14.07.2022 Gazette 2022/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.01.2021 KR 20210002116**

(71) Applicant: **Lg Chem, Ltd.**
**Seoul 07336 (KR)**

(72) Inventors:
- **JUNG, Hyun Tae**
  **Daejeon 34122 (KR)**
- **LEE, Chung**
  **Daejeon 34122 (KR)**
- **SO, Jineon**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

# (54) CROSSLINKED HYALURONIC ACID HYDROGEL CROSSLINKED USING CROSSLINKER AND POLYOL, AND FILLER COMPRISING SAME

(57) The present invention provides a crosslinked hyaluronic acid hydrogel in which a crosslinking agent and a polyol are used to reduce toxicity during crosslinking, and thereby enhance safety while increasing persistence in the body; and a filler composition including the crosslinked hyaluronic acid hydrogel.

【FIG. 2】

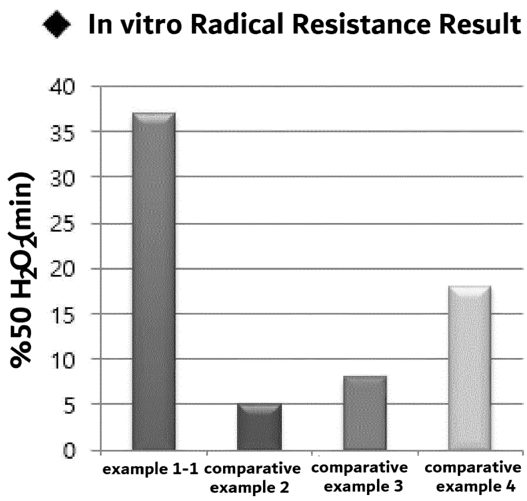


EP 4 245 328 A1

## Description

[TECHNICAL FIELD]

The present invention relates to a hyaluronic acid hydrogel crosslinked using a polyol with a crosslinking agent and a filler comprising thereof.

[BACKGROUND OF THE INVENTION]

Fillers developed for wrinkle improvement widely use hyaluronic acid, a natural polymer with high biocompatibility. In order to increase the duration of hyaluronic acid in the body, a hyaluronic acid hydrogel crosslinked using various crosslinking agents have beencurrently used for fillers. However, since the crosslinking agent is generally highly toxic, there is a disadvantage that it is difficult to use a large amount. In addition, when the crosslinking ratio is low in spite of crosslinked hydrogel, there is a disadvantage of rapidly decomposing by hyaluronidase or reactive oxygen species (radicals), so it was difficult to increase both stability and durability in the body.

[DISCLOSURE]

[TECHNICAL PROBLEM]

As a result of repeated research to solve problems of the prior art as described above, the present inventors have confirmed that when hyaluronic acid is crosslinked using a polyol such as sugar alcohol together with a conventionally used crosslinking agent, the stability to hyaluronidase, reactive oxygen species (radicals) and/or heat is excellently improved, and also, the toxicity is significantly reduced due to use of a small amount of crosslinking agent, and therefore a filler with high biocompatibility can be prepared, thereby completing the present invention.

Accordingly, an object of the present invention is to provide a crosslinked hyaluronic acid hydrogel which reduces toxicity during crosslinking to have high safety and high durability in the body and a composition for a filler comprising thereof.

[TECHNICAL SOLUTION]

According to one aspect of the present invention, a crosslinked hyaluronic acid hydrogel comprising hyaluronic acid or its salt, a crosslinking agent and a polyol, in which the hyaluronic acid or its salt is crosslinked with the crosslinking agent and polyol, and a filler comprising the crosslinked hyaluronic acid hydrogel.

According to one aspect of the present invention, the filler is for soft tissue injection, for example, for skin injection, and the filler may be used for filling properties, for example, filler uses such as filling of biological tissue, wrinkle improvement of filling wrinkle, remodeling of the face or contour correction, or volume repair or increase of soft tissue.

According to one aspect of the present invention, the polyol may be sugar alcohol.

According to one aspect of the present invention, a method for preparation of the crosslinked hyaluronic acid hydrogel comprising mixing hyaluronic acid or its salt with a polyol and adding a mixed solution of a crosslinking agent and an aqueous alkali solution into the mixture of the hyaluronic acid or its salt and polyol and reacting may be provided.

According to one aspect of the present invention, a prefilled syringe filled with a filler comprising the crosslinked hyaluronic acid is provided.

[ADVANTAGEOUS EFFECTS]

Resistance to hyaluronidase, reactive oxygen species in the body and storage temperature is increased, since the crosslinked hyaluronic acid hydrogel according to the present invention uses a crosslinking agent with a polyol. Therefore, the filler comprising this crosslinked hyaluronic acid hydrogel has increased stability and reduced toxicity during the storage period to exhibit high safety and have excellent durability in the body, and thus soft tissue repair or volume enlargement and wrinkle improvement effects are excellent.

[BRIEF DESCRIPTION OF THE DRAWINGS]

FIG. 1 is a graph of comparing the in vitro enzyme resistance result of the crosslinked hyaluronic acid hydrogel crosslinked using a crosslinking agent and mannitol of Example 1-1 according to the present invention with Comparative examples 2 to 4.

FIG. 2 is a graph of comparing the in vitro radical resistance result of the crosslinked hyaluronic acid hydrogel crosslinked using a crosslinking agent and mannitol of Example 1-1 according to the present invention with Comparative examples 2 to 4.

FIG. 3 is a graph of comparing the in vitro heat resistance result of the crosslinked hyaluronic acid hydrogel crosslinked using a crosslinking agent and mannitol of Example 1-1 according to the present invention with Comparative examples 2 to 4.

FIG. 4 is a graph of comparing the in vivo biocompatibility test result of the crosslinked hyaluronic acid hydrogel crosslinked using a crosslinking agent and mannitol of Example 1-1 according to the present invention with Comparative examples 2 to 4.

[BEST MODE]

Hereinafter, the present invention will be described in detail.

Hyaluronic acid (hereinafter, also called 'HA') contained in the crosslinked hyaluronic acid hydrogel of the present invention is a biopolymer substance in which disaccharide repeating units (disaccharide units) consisting of N-acetyl-D-glucosamine and D-glucuronic acid are linearly connected, and is abundantly present in the vitreous humor of the eye, synovial fluid of joints, cockscomb, and the like, and has excellent biocompatibility, and thus, it is widely used for medical treatment and medical devices or cosmetic uses such as ophthalmic surgical aids, viscosupplementation for joint, drug delivery materials, eye drops, wrinkle improving agents, and the like.

Specifically, hyaluronic acid contained in the hyaluronic acid hydrogel according to the present invention may refer to hyaluronic acid or a salt of hyaluronic acid. The salt of hyaluronic acid includes, for example, all of organic salts such as sodium hyaluronate, potassium hyaluronate, calcium hyaluronate, magnesium hyaluronate, zinc hyaluronate, cobalt hyaluronate and tetrabutylammonium hyaluronate, but not limited thereto.

In the present invention, the weight average molecular weight of hyaluronic acid used for the crosslinking reaction may be 1,000,000 Da or more, 1,500,000 Da or more, 2,000,000 Da or more, 2,300,000 Da or more, or 2,500,000 Da or more, and for example, it is 1,000,000 to 1,500,000 Da, 1,000,000 to 2,000,000 Da, 1,000,000 to 3,000,000 Da, 1,000,000 to 4,000,000 Da, 1,500,000 to 2,000,000 Da, 1,500,000 to 3,000,000 Da, 1,500,000 to 4,000,000 Da, 2,000,000 to 4,000,000 Da, 2,300,000 to 4,000,000 Da, 2,000,000 to 3,700,000 Da, 2,200,000 to 3,700,000 Da, or 2,500,000 to 3,500,000 Da.

The crosslinked hyaluronic acid hydrogel according to the present invention is characterized in that the hyaluronic acid or its salt is crosslinked using a crosslinking agent and a polyol.

The term "crosslinked" used in the present invention refers to an intermolecular bond which binds individual polymer molecules or monomer chains into a more stable structure such as gel. As such, a crosslinked polymer has at least one intermolecular bond linking at least one individual polymer molecule to another.

The crosslinked hyaluronic acid hydrogel according to the present invention is characterized in that the hyaluronic acid or its salt is crosslinked using a crosslinking agent and a polyol, different from a conventional hyaluronic acid hydrogel crosslinked only with a crosslinking agent.

The term "crosslinking agent" means any compound capable of inducing crosslinking between hyaluronic acid chains, and in the present invention, the crosslinking agent may be used without limitation, which can crosslink hyaluronic acid or its salt, and for example, it may vary, for example, as a compound comprising two or more epoxy functional groups. As a preferable example of the crosslinking agent may include endogenous polyamine, aldehyde, carbodiimide, divinyl sulfone as a non-epoxy crosslinking agent. In addition, the epoxy crosslinking agent may include butanediol diglycidyl ether (1,4-butanediol diglycidyl ether: BDDE), ethylene glycol diglycidyl ether (EGDGE), hexanediol diglycidyl ether (1,6-hexanediol diglycidyl ether), propylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, tri-methylpropane polyglycidyl ether, bisepoxypropoxy ethylene (1,2-(bis(2,3-epoxypropoxy)ethylene), pentaerythritol polyglycidyl ether and sorbitol polyglycidyl ether, and the like, and among them, biepoxide-based 1,4-butanediol diglycidyl ether is particularly preferable in view of low toxicity.

The polyol which crosslinks hyaluronic acid or its acceptable salt with the crosslinking agent is linked with the crosslinking agent to crosslink hyaluronic acid or its acceptable salt. This polyol refers to an organic molecule comprising 2 or more free hydroxy groups, and it may be a polyol having 2 to 20 carbon atoms, more specifically, sugar alcohol. The polyol suitable for the present invention may be a saturated or unsaturated, linear, branched or cyclic alkyl-bearing compound, which has at least 2 -OH functional groups, for example, 3 or more -OH functional groups and more specifically, 4 or more -OH functional groups, on its alkyl chain. Non-limitative examples of the polyol are as follows: glycerin, 1,3-propanediol, isoprene glycol, pentylene glycol, hexylene glycol; glycol such as ethylene glycol, propylene glycol, butylene glycol, diethylene glycol; polyglycerol of 2 to 6 repeating units, for example, diglycerol, erythritol, arabitol, adonitol, sorbitol, mannitol, xylitol, dulcitol, glucose, fructose, xylose, trehalose, maltose, saccharose, lactose; and derivatives

thereof and mixtures thereof such as methylglucoside phosphate. More specifically, the polyol may be selected from the group consisting of mannitol, sorbitol and xylitol. In the present invention, in the polyol, the -OH functional group reacts with reactive oxygen species to make the reactive oxygen species inactive, thereby inhibiting decomposition of a filler by reactive oxygen species.

As one specific example, the hyaluronic acid or its acceptable salt may be linked with a crosslinking agent and a polyol as follows.

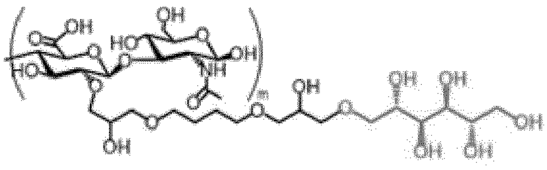

HA-BDDE-Mannitol

In other words, the hydroxy group of hyaluronic acid or its acceptable salt is linked to one of epoxide groups at both ends of the crosslinking agent, and the epoxide group of the other end of the crosslinking agent and the hydroxy group of the polyol are linked to be a form of hyaluronic acid-crosslinking agent-polyol. Furthermore, another hydroxy group of the polyol is continuously linked to the hydroxy group of new hyaluronic acid or its acceptable salt as follows and eventually, a crosslinked hyaluronic acid hydrogel is prepared.

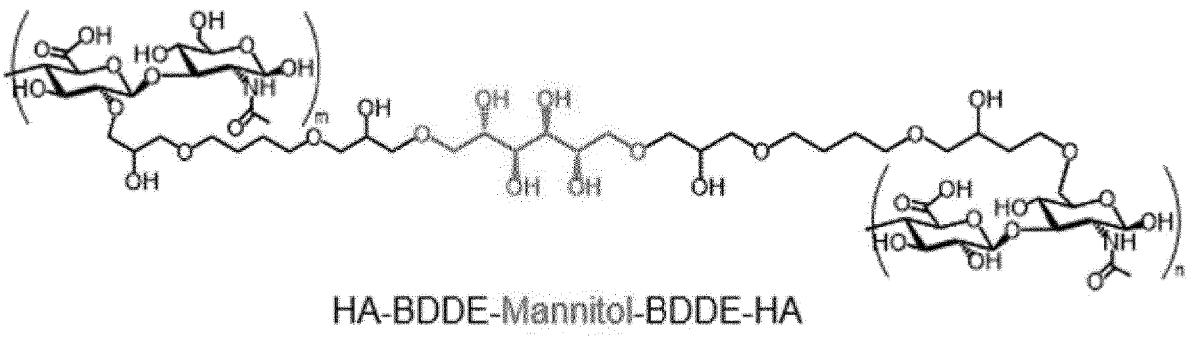

HA-BDDE-Mannitol-BDDE-HA

The crosslinked hyaluronic acid hydrogel according to the present invention has a modification degree (MoD) in a range of about 3 to about 100, preferably, about 3 to about 80, more preferably, about 3 to about 50.

In addition, in the present invention, the term "modification degree (MoD)" refers to a molar ratio of the total crosslinking agent linked to hyaluronic acid to the number of moles of the total unit hyaluronic acid, and it may be expressed as in Equation 1 below.

[Equation 1]

Modification degree (MoD) = Number of moles of crosslinking agent linked to hyaluronic acid / number of moles of unit hyaluronic acid

The crosslinked hyaluronic acid hydrogel according to the present invention may comprise the total hyaluronic acid of about 10 mg/g to about 40 mg/g, preferably, about 15 mg/g to about 35 mg/g, much more preferably, about 20 mg/g to about 30 mg/g, based on the total weight of the crosslinked hyaluronic acid hydrogel.

The crosslinked hyaluronic acid hydrogel according to the present invention exhibits the effect of replacing decomposition of hyaluronic acid by reactive oxygen species in which a polyol chemically combined to hyaluronic acid or its salt, and structurally interferes with the mechanism of binding and decomposition of hyaluronidase, and thereby, it has an advantage in that when a filler comprising a crosslinked hyaluronic acid hydrogel is injected into the human body, the effect of inhibiting decomposition of the filler is exhibited, so that the durability is increased after injection into the body. In addition, the crosslinked hyaluronic acid hydrogel may be decomposed as the β-1-4-glycosidic bond is broken in which the chemical bond between hyaluronic acid molecules is relatively weak by not only the enzyme as

above but also heat, and according to the present invention, the polyol has thermal stability that inhibits thermal decomposition of such hyaluronic acid and has an advantage during storage, so it is very useful as a filler.

As another aspect, the present invention relates to a method for preparation of the crosslinked hyaluronic acid hydrogel using a crosslinking agent and a polyol. Specifically, the method for preparation of a crosslinked hyaluronic acid hydrogel according to the present invention, may comprise (i) mixing hyaluronic acid or its salt with a polyol; and (ii) adding a mixed solution of a crosslinking agent and an aqueous alkali solution into the mixture of the hyaluronic acid or its salt and polyol and reacting.

In the method for preparation, for matters relating to hyaluronic acid or its salt, crosslinking agent and polyol, those relating to the crosslinked hyaluronic acid hydrogel may be equally applied unless otherwise stated.

In addition, the weight average molecular weight of the hyaluronic acid or its salt may be 1,000,000 Da or more, 1,500,000 Da or more, 2,000,000 Da or more, 2,300,000 Da or more, or 2,500,000 Da or more, and for example, it is 1,000,000 to 1,500,000 Da, 1,000,000 to 2,000,000 Da, 1,000,000 to 3,000,000 Da, 1,000,000 to 4,000,000 Da, 1,500,000 to 2,000,000 Da, 1,500,000 to 3,000,000 Da, 1,500,000 to 4,000,000 Da, 2,000,000 to 4,000,000 Da, 2,300,000 to 4,000,000 Da, 2,000,000 to 3,700,000 Da, 2,200,000 to 3,700,000 Da, or 2,500,000 to 3,500,000 Da.

On the other hand, the concentration of the polyol used in the method for preparation may be 5 to 100 mol%, 10 to 100mol%, 10 to 90mol% or 10 to 80mol% based on hyaluronic acid or its salt, but not limited thereto, and may be appropriately adjusted depending on reaction conditions.

In addition, the aqueous alkali solution may be used without limitation as long as it is known as an aqueous alkali solution suitable for crosslinking of hyaluronic acid, and for example, it may be NaOH, KOH, NaHCOs, LiOH or a combination thereof, and preferably, it may be NaOH. The concentration of the aqueous alkali solution may be 0.1 to 0.5N, but not limited thereto. The concentration of the aqueous alkali solution used in the method for preparation may be 5 to 100 mol%, 10 to 100mol%, 10 to 90mol% or 10 to 80mol% based on hyaluronic acid or its salt, but not limited thereto, and it may be appropriately adjusted depending on reaction conditions.

When the concentration of the crosslinking agent and/or polyol is used at a high concentration exceeding the above range, a filler with excessively high elasticity is obtained, and when the concentration is less than the above range, the elasticity is too low to exhibit proper viscoelasticity. Specifically, the crosslinking reaction may be carried out by stirring a mixture of hyaluronic acid or its salt and a polyol, and a crosslinking agent and an aqueous alkali solution to homogeneously mix them, and then maintaining them for a certain period of time. The temperature during the crosslinking reaction may be a room temperature or more, preferably, in the temperature range of 25 to 65°C or 27 to 55°C for 1 to 22 hours or 2 to 20 hours.

In the method for preparation according to the present invention, the total reaction concentration (that is, a ratio of the sum weight of the hyaluronic acid and polyol to the total weight of the hyaluronic acid, polyol and solvent) may be 5 to 30% (w/w), or 10 to 30% (w/w).

In one specific example of the present invention, a substance was prepared by mixing mannitol, sorbitol or xylitol of 10mol% based on hyaluronic acid with hyaluronic acid, mixing a mixed solution of NaOH solution and 1,4-BDDE and maintaining at 30 to 50°C for 2 hours, and then washing/neutralizing/swelling crosslinked gel, and then pulverizing it using a mesh net and then sterilizing.

In other aspect, the present invention relates to a filler comprising the crosslinked hyaluronic acid hydrogel.

The filler may comprise a crosslinked hyaluronic acid hydrogel in an amount of 0.5 to 10 % by weight, preferably, 1 to 5 % by weight based on the total filler weight.

Furthermore, the filler comprising a crosslinked hyaluronic acid hydrogel according to the present invention may further comprise an anesthetic to reduce pains of a patient during injection, in addition to the crosslinked hyaluronic acid hydrogel.

The anesthetic includes at least one anesthetic known in the art, preferably, local anesthetic, and the concentration of such one or more anesthetics is an amount effective to relieve pains experienced when a composition is injected. The example of the anesthetic may be selected from the group consisting of ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dycyclonine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, psuedococaine, pyrrocaine, ropivacaine, salicyl alcohol, tetracaine, tolycaine, trimecaine, zolamine, and salts thereof. In one embodiment, the anesthetic may be lidocaine, for example, in a form of lidocaine hydrochloride.

In the filler comprising a crosslinked hyaluronic acid hydrogel according to the present invention, the concentration of the anesthetic comprised in the filler may be about 0.1 % by weight to about 1.0 % by weight based on the total weight of the filler, for example, about 0.2% by weight to about 0.5% by weight of the composition. It may be

preferably 0.3 % by weight.

The concentration of the anesthetic in the filler according to the present invention may be therapeutically effective, and this means a concentration suitable for providing advantages in terms of convenience of a procedure and patient compliance without harming the patient.

In addition, the filler according to the present invention may further comprise a buffer solution, and the buffer solution may be used without limitation as long as it is used for preparation of a hyaluronic acid hydrogel. The example of the preferable buffer solution may include a buffer solution comprising one or more kinds selected from the group consisting of citric acid, sodium monohydrogen phosphate, sodium dihydrogen phosphate, diethyl barbituric acid, sodium acetate, TAPS (tris(hydroxymethyl)methylamino)propane sulfonate), Bicine (2-bis(2-hydroxyethyl)amino)acetate), Tris (tris(hydroxymethyl)ammonium methane), Tricine (N-(2-hydroxy-1, 1-bis(hydroxymethyl)ethyl)glycine), HEPES (4-(2-hydroxyethyl)-1-piperazine ethane sulfonate), TES (2-[[1,3-dihydroxy-2-(hydroxymethyl)propan-2-yl]amino]methane sulfonate) and PIPES (piperazine-N,N'-bis(2-ethane sulfonate), but not limited thereto. The content of the above components comprised in the buffer solution may be appropriately adjusted, but preferably, it may be comprised at a concentration of 0.3 to 2.0g/L based on the buffer solution.

In addition, the filler according to the present invention may further comprise an isotonic agent, and this isotonic agent may be used without limitation as long as it is used for preparation of a filler, and it may be comprised in the buffer solution. As a preferable isotonic agent, sodium chloride may be used, but not limited thereto. The content of the isotonic agent may be appropriately adjusted if needed, and for example, it may be comprised in 7.0 to 9.0g/L based on the buffer solution, but not limited thereto.

In one example according to the present invention, a buffer solution comprising sodium chloride, sodium monohydrogen phosphate and sodium dihydrogen phosphate in water for injection was used.

As an additional aspect, the filler comprising a crosslinked hyaluronic acid hydrogel according to the present invention may further comprise acceptable components comprised in preparation of a filler, in addition to the above component.

The filler comprising a crosslinked hyaluronic acid hydrogel according to the present invention has an advantage that the storage period may be significantly increased compared to conventional hyaluronic acid filler formulations due to high thermal stability. In addition, it has high resistance to degradation by hyaluronidase and reactive oxygen species (radicals), so it can significantly increase the duration after injection into the human body. As it exhibits very excellent properties in terms of biocompatibility and toxicity, it may be very usefully used for cosmetic or therapeutic purposes.

As one specific aspect, the filler comprising a crosslinked hyaluronic acid hydrogel according to the present invention may be used for wrinkle improvement by filling of biological tissue and filling wrinkle, remodeling of the face or repair or increase of the volume of soft tissue such as lip, nose, hip, cheek or breast, or the like. The filler comprising a hyaluronic acid hydrogel may be administered in an administration form suitable for such a use, and it may be preferably an injection, more preferably a pre-filled injection (pre-filled syringe).

As other aspect, the present invention relates to a method for preparation of a filler comprising the crosslinked hyaluronic acid hydrogel as above comprising the following steps:

(a) mixing hyaluronic acid or its salt with a polyol;
(b) adding a mixed solution of a crosslinking agent and an aqueous alkali solution into the mixture of the hyaluronic acid or its salt and polyol and reacting to prepare a crosslinked hyaluronic acid hydrogel;
(c) crude cutting the hyaluronic acid hydrogel prepared in the (b);
(d) washing and swelling the crude cut hyaluronic acid hydrogel prepared in the (b) using a buffer solution; and
(e) pulverizing the washed and swelled hyaluronic acid hydrogel in the (d).

The steps (a) and (b) are steps of preparing a crosslinked hyaluronic acid hydrogel by crosslinking hyaluronic acid or its salt using a crosslinking agent and a polyol on an aqueous alkali solution, and to the matters relating to the hyaluronic acid or its salt, crosslinking agent, polyol and crosslinked hyaluronic acid hydrogel, those mentioned in the crosslinked hyaluronic acid hydrogel and method for preparation thereof may be equally applied.

In the crude cutting process (step (c)), various crude cutting processes of hyaluronic acid hydrogel may be used. In one example, the crosslinked hyaluronic acid hydrogel prepared after reaction may be obtained in the shape of a cake (or cylinder) and this may be divided into a half-moon shape, for example, into 6 parts by using a cutter such as a straw cutter. Then, the crude cutting process may be performed by passing gel divided as described above (preferably two or more times) using a cutter having a constant blade interval.

It may be prepared according to known buffer solution preparation methods used in the step (d). In addition, in the buffer solution, an anesthetic may be further comprised additionally. The buffer solution may be used without limitation as long as it is used for preparation of hyaluronic acid hydrogel. As the example of this preferable buffer solution, a buffer solution comprising one or more kinds selected from the group consisting of citric acid, sodium monohydrogen phosphate, sodium dihydrogen phosphate, diethyl barbituric acid, sodium acetate, TAPS (tris(hydroxymethyl)methylamino)propane sulfonate), Bicine (2-bis(2-hydroxyethyl)amino)acetate), Tris (tris(hydroxymethyl)ammonium methane), Tricine (N-(2-hydroxy-1,1-bis(hydroxymethyl)ethyl)glycine), HEPES (4-(2-hydroxyethyl)-1-piperazine ethane sulfonate), TES (2-[[1,3-dihycroxy-2-(hydroxymethyl)propan-2-yl]amino]methane sulfonate) and PIPES (piperazine-N,N'-bis(2-ethane sulfonate), but not limited thereto.

In addition, washing and swelling may be repeated once or twice. When washing and swelling are completed, the washing solution may be removed.

The step (e) is a step of pulverizing washed and swelled hydrogel, and this pulverizing may be performed by various pulverizing methods, but preferably, it is extrusion pulverizing.

As an additional aspect, the crosslinked hydrogel filler prepared after the step (e) may pass through a process of sterilization and/or defoaming, and the like, and it may be filled, sealed and sterilized in quantity in an appropriate container, for example, a prefilled syringe.

[MODE FOR INVENTION]

Hereinafter, to help understanding of the present invention, it will be described in detail with examples. However, the following examples are intended to illustrate the contents of the present invention only, but the scope of the present invention is not limited by the following examples. The examples of the present invention are provided to more completely explain the present invention to those skilled in the art.

**[Example]**

**Example 1-1: Preparation of crosslinked hyaluronic acid hydrogel crosslinked with mannitol and crosslinking agent according to the present invention (1)**

In order to prepare a crosslinked hyaluronic acid hydrogel crosslinked with mannitol and a crosslinking agent according to the present invention, the following process was conducted.

Specifically, hyaluronic acid sodium salt having an average molecular weight of 3 million Da, sodium hydroxide, BDDE (1,4-butanediol diglycidyl ether) as a crosslinking agent, and mannitol were weighed, respectively.

Hyaluronic acid 1g was weighed and 10 mol% of mannitol based on the hyaluronic acid weight was weighed, and then they were put in a mixer container and mixed. Sodium hydroxide (NaOH) aqueous solution at a concentration of 0.25N was added to a separate container (50mL tube) so that the total reaction concentration was 10% (w/w) based on the hyaluronic acid weight, and 1,4-butanediol diglycidyl ether (BDDE) of 100mol% based on the hyaluronic acid weight was added and mixed. The mixture contained in the container was put into a mixer container in which hyaluronic acid and mannitol were mixed and mixed using a mixer, and then the mixer container was put in a constant-temperature water bath and crosslinking was completed while maintain at 50°C for 2 hours. Then, the crosslinked hyaluronic acid hydrogel obtained after completing the reaction was crude cut into a certain size, and using buffer solution (buffer solution prepared by dissolving sodium monohydrogen phosphate hydrate (dodecahydrate) 1.26g/L, sodium dihydrogen phosphate hydrate (monohydrate) 0.46g/L, sodium chloride 7g/L, and lidocaine hydrochloride 3g/L in a 500ml bottle container containing water for injection), it was washed and swelled 6 times for 1 hour each. After pulverizing hyaluronic acid hydrogel in which washing and swelling was completed, it was moved into a 250 ml bottle container and the weight was measured, and the buffer solution was added so that the gel weight reached a target weight, and thereby, primary content correction was conducted. When the primary content correction was completed, hyaluronic acid hydrogel was extruded from the 250ml bottle container and pulverized using a mesh net. Then, the pulverized hyaluronic acid hydrogel was transferred into a 250ml bottle container and homogenized, and then the content was measured and the buffer solution was added, and thereby secondary content correction was conducted. The hyaluronic acid hydrogel in which the content correction was completed was sterilized by heat treatment at a temperature of 121°C or more for 10 minutes or more to prepare the crosslinked hyaluronic acid hydrogel according to the present invention.

**Example 1-2: Preparation of crosslinked hyaluronic acid hydrogel crosslinked with mannitol and crosslinking agent according to the present invention (2)**

In order to prepare a crosslinked hyaluronic acid hydrogel crosslinked with mannitol and a crosslinking agent according to the present invention, the following process was conducted.

Specifically, hyaluronic acid sodium salt having an average molecular weight of 3 million Da, sodium hydroxide, BDDE (1,4-butanediol diglycidyl ether) as a crosslinking agent, and mannitol were weighed, respectively.

Hyaluronic acid 2g was weighed and 10 mol% of mannitol based on the hyaluronic acid weight was weighed, and then they were put in a mixer container and mixed. Sodium hydroxide (NaOH) aqueous solution at a concentration of 0.25N was added to a separate container (50mL tube) so that the total reaction concentration was 15% (w/w) based

on the hyaluronic acid weight, and 1,4-butanediol diglycidyl ether (BDDE) of 5mol% based on the hyaluronic acid weight was added and mixed. The mixture contained in the container was put into a mixer container in which hyaluronic acid and mannitol were mixed and mixed using a mixer, and then the mixer container was put in a constant-temperature water bath and crosslinking was completed while maintain at 30°C for 19 hours. Then, the crosslinked hyaluronic acid hydrogel obtained after completing the reaction was crude cut into a certain size, and using buffer solution (buffer solution prepared by dissolving sodium monohydrogen phosphate hydrate (dodecahydrate) 1.26g/L, sodium dihydrogen phosphate hydrate (monohydrate) 0.46g/L, sodium chloride 7g/L, and lidocaine hydrochloride 3g/L in a 500ml bottle container containing water for injection), it was washed and swelled once for 2 hours and twice for 1 hour. After pulverizing hyaluronic acid hydrogel in which washing and swelling was completed, it was moved into a 150 ml bottle container and the weight was measured, and the buffer solution was added so that the gel weight reached a target weight, and thereby, primary content correction was conducted. When the primary content correction was completed, hyaluronic acid hydrogel was extruded from the 150ml bottle container and pulverized using a mesh net. Then, the pulverized hyaluronic acid hydrogel was transferred into a 150ml bottle container and homogenized, and then the content was measured and the buffer solution was added, and thereby secondary content correction was conducted. The hyaluronic acid hydrogel in which the content correction was completed was sterilized by heat treatment at a temperature of 121°C or more for 10 minutes or more to prepare the crosslinked hyaluronic acid hydrogel according to the present invention.

## Example 2: Preparation of crosslinked hyaluronic acid hydrogel crosslinked with sorbitol and crosslinking agent according to the present invention

In order to prepare a crosslinked hyaluronic acid hydrogel crosslinked with sorbitol and a crosslinking agent according to the present invention, the following process was conducted.

Specifically, hyaluronic acid sodium salt having an average molecular weight of 3 million Da, sodium hydroxide, BDDE (1,4-butanediol diglycidyl ether) as a crosslinking agent, and sorbitol were weighed, respectively.

Hyaluronic acid 2g was weighed and 10 mol% of sorbitol based on the hyaluronic acid weight was weighed, and then they were put in a mixer container and mixed. Sodium hydroxide (NaOH) aqueous solution at a concentration of 0.25N was added to a separate container (50mL tube) so that the total reaction concentration was 15% (w/w) based on the hyaluronic acid weight, and 1,4-butanediol diglycidyl ether (BDDE) of 5mol% based on the hyaluronic acid weight was added and mixed. The mixture contained in the container was put into a mixer container in which hyaluronic acid and sorbitol were mixed and mixed using a mixer, and then the mixer container was put in a constant-temperature water bath and crosslinking was completed while maintain at 30°C for 19 hours. Then, the crosslinked hyaluronic acid hydrogel obtained after completing the reaction was crude cut into a certain size, and using buffer solution (buffer solution prepared by dissolving sodium monohydrogen phosphate hydrate (dodecahydrate) 1.26g/L, sodium dihydrogen phosphate hydrate (monohydrate) 0.46g/L, sodium chloride 7g/L, and lidocaine hydrochloride 3g/L in a 500ml bottle container containing water for injection), it was washed and swelled once for 2 hours and twice for 1 hour. After pulverizing hyaluronic acid hydrogel in which washing and swelling was completed, it was moved into a 150 ml bottle container and the weight was measured, and the buffer solution was added so that the gel weight reached a target weight, and thereby, primary content correction was conducted. When the primary content correction was completed, hyaluronic acid hydrogel was extruded from the 150ml bottle container and pulverized using a mesh net. Then, the pulverized hyaluronic acid hydrogel was transferred into a 150ml bottle container and homogenized, and then the content was measured and the buffer solution was added, and thereby secondary content correction was conducted. The hyaluronic acid hydrogel in which the content correction was completed was sterilized by heat treatment at a temperature of 121°C or more for 10 minutes or more to prepare the crosslinked hyaluronic acid hydrogel according to the present invention.

## Example 3: Preparation of crosslinked hyaluronic acid hydrogel crosslinked with xylitol and crosslinking agent according to the present invention

In order to prepare a crosslinked hyaluronic acid hydrogel crosslinked with xylitol and a crosslinking agent according to the present invention, the following process was conducted.

Specifically, hyaluronic acid sodium salt having an average molecular weight of 3 million Da, sodium hydroxide, BDDE (1,4-butanediol diglycidyl ether) as a crosslinking agent, and xylitol were weighed, respectively.

Hyaluronic acid 2g was weighed and 10 mol% of xylitol based on the hyaluronic acid weight was weighed, and then they were put in a mixer container and mixed. Sodium hydroxide (NaOH) aqueous solution at a concentration of 0.25N was added to a separate container (50mL tube) so that the total reaction concentration was 15% (w/w) based on the hyaluronic acid weight, and 1,4-butanediol diglycidyl ether (BDDE) of 5mol% based on the hyaluronic acid weight was added and mixed. The mixture contained in the container was put into a mixer container in which hyaluronic acid and xylitol were mixed and mixed using a mixer, and then the mixer container was put in a constant-temperature water bath and crosslinking was completed while maintain at 30°C for 19 hours. Then, the crosslinked hyaluronic acid hydrogel

obtained after completing the reaction was crude cut into a certain size, and using buffer solution (buffer solution prepared by dissolving sodium monohydrogen phosphate hydrate (dodecahydrate) 1.26g/L, sodium dihydrogen phosphate hydrate (monohydrate) 0.46g/L, sodium chloride 7g/L, and lidocaine hydrochloride 3g/L in a 500ml bottle container containing water for injection), it was washed and swelled once for 2 hours and twice for 1 hour. After pulverizing hyaluronic acid hydrogel in which washing and swelling was completed, it was moved into a 150 ml bottle container and the weight was measured, and the buffer solution was added so that the gel weight reached a target weight, and thereby, primary content correction was conducted. When the primary content correction was completed, hyaluronic acid hydrogel was extruded from the 150ml bottle container and pulverized using a mesh net. Then, the pulverized hyaluronic acid hydrogel was transferred into a 150ml bottle container and homogenized, and then the content was measured and the buffer solution was added, and thereby secondary content correction was conducted. The hyaluronic acid hydrogel in which the content correction was completed was sterilized by heat treatment at a temperature of 121°C or more for 10 minutes or more to prepare the crosslinked hyaluronic acid hydrogel according to the present invention.

**Comparative example 1: Preparation of crosslinked hyaluronic acid hydrogel crosslinked with crosslinking agent according to conventional method**

In order to prepare a crosslinked hyaluronic acid hydrogel crosslinked with a crosslinking agent according to a conventional method, the following process was conducted.

Specifically, hyaluronic acid sodium salt having an average molecular weight of 3 million Da, sodium hydroxide, and BDDE (1,4-butanediol diglycidyl ether) as a crosslinking agent were weighed, respectively.

Hyaluronic acid 2g was weighed and sodium hydroxide (NaOH) aqueous solution at a concentration of 0.25N was added to a separate container (50mL tube) so that the total reaction concentration was 15% (w/w) based on the hyaluronic acid weight, and 1,4-butanediol diglycidyl ether (BDDE) of 5mol% based on the hyaluronic acid weight was added and mixed. The mixture contained in the container was put into a mixer container in which hyaluronic acid was mixed and mixed using a mixer, and then the mixer container was put in a constant-temperature water bath and crosslinking was completed while maintain at 30°C for 19 hours. Then, the crosslinked hyaluronic acid hydrogel obtained after completing the reaction was crude cut into a certain size, and using buffer solution (buffer solution prepared by dissolving sodium monohydrogen phosphate hydrate (dodecahydrate) 1.26g/L, sodium dihydrogen phosphate hydrate (monohydrate) 0.46g/L, sodium chloride 7g/L, and lidocaine hydrochloride 3g/L in a 500ml bottle container containing water for injection), it was washed and swelled once for 2 hours and twice for 1 hour. After pulverizing hyaluronic acid hydrogel in which washing and swelling was completed, it was moved into a 150 ml bottle container and the weight was measured, and the buffer solution was added so that the gel weight reached a target weight, and thereby, primary content correction was conducted. When the primary content correction was completed, hyaluronic acid hydrogel was extruded from the 150ml bottle container and pulverized using a mesh net. Then, the pulverized hyaluronic acid hydrogel was transferred into a 150ml bottle container and homogenized, and then the content was measured and the buffer solution was added, and thereby secondary content correction was conducted. The hyaluronic acid hydrogel in which the content correction was completed was sterilized by heat treatment at a temperature of 121°C or more for 10 minutes or more to prepare the crosslinked hyaluronic acid hydrogel according to the present invention.

**Comparative examples 2, 3, 4, 5, 6 and 7**

Commercially available dermal fillers A, B, C, D, E and F were tested as Comparative examples 2 to 7, respectively.

[Fillers of Comparative examples 2-7]

A: Juvederm voluma lidocaine, Allergan
B: Restylane lidocaine, Galderma
C: YVOIRE volume s, LG Chem
D: Ellanse, Sinclair
E: Cleviel, Pharma Research Products
F: YVOIRE classic plus, LG Chem

**Experimental example 1: Investigation of viscoelastic properties of crosslinked hyaluronic acid hydrogel prepared according to the present invention**

For investigation of rheological properties of prepared Examples 1-2, 2 and 3 and Comparative example 1, they were analyzed using a rheometer. The analysis condition is as follows.

<Analysis condition>

Analysis condition of Oscillatory and Rotational Rheometer

In case of complex viscosity (η*) test

(1) Test equipment: Rheometer (Anton Paar Ltd., MCR301)
(2) Frequency: 1 Hz
(3) Temperature: 25 °C
(4) Strain: 4 %
(5) Measuring geometry: 25 mm plate
(9) Measuring gap: 1.0 mm

The analysis result was shown in Table 1.

[Table 1]

| Sample | Complex viscosity (x$10^4$ cP) |
|---|---|
| Example 1-2 | 380 |
| Example 2 | 381 |
| Example 3 | 410 |
| Comparative example 1 | 370 |

As the result of the complex viscosity analysis of each sample, it was confirmed that the complex viscosity of Examples 1-2, 2 and 3 which were crosslinked hyaluronic acid hydrogel prepared using mannitol, sorbitol and xylitol that were sugar alcohols was higher than Comparative example 1 prepared using only a crosslinking agent.

Experimental example 2-1: ***In vitro* enzyme resistance investigation (1)**

When crosslinked hyaluronic acid (HA) hydrogel is injected into the body, this crosslinked hyaluronic acid hydrogel is subjected to a direct decomposition attacked by hyaluronidase to be decomposed. By directly injecting hyaluronidase into a sample, it is possible to check degradation tendency and measure the resistance to the enzyme. Specifically, the enzyme resistance test was performed in situ according to the following method using a rheometer.

After pulverizing the crosslinked hyaluronic acid hydrogel prepared by Examples 1-2, 2 and 3 and the crosslinked hyaluronic acid hydrogel of Comparative example 1, respectively, and then finally sterilizing, the sterilized sample 1g was weighed to a 50mL tube, and then hyaluronidase 200uL prepared with 500unit/mL was added and mixed. The sample mixed with the enzyme was loaded on the rheometer and the temperature was set to 37°C, and then the complex viscosity of the sample was measured in real time. The higher the hyaluronidase resistance, the higher the residual rate of the complex viscosity compared to the initial value of the complex viscosity. It means that the higher the residual rate of the complex viscosity, the higher the resistance to the enzyme. Based on the measured result, %50 Hase (min) was calculated and the result was shown in Table 2. The %50 Hase (min) means time it takes for the complex viscosity of crosslinked hyaluronic acid hydrogel to decrease to 50% of its initial physical properties due to enzyme decomposition. In other words, it means that the higher the value of %50 Hase (min), the higher the resistance of the crosslinked hyaluronic acid hydrogel.

[Table 2]

| Sample | %50 Hase (Minute) |
|---|---|
| Example 1-2 | 42 |
| Example 2 | 45 |
| Example 3 | 41 |
| Comparative example 1 | 33 |

As confirmed in Table 2, the %50 Hase (min) of Examples 1 to 3 for crosslinked hyaluronic acid hydrogel comprising sugar alcohol according to the present invention was 42 minutes, 45 minutes and 41 minutes, respectively, while Comparative example 1 that was the crosslinked hyaluronic acid hydrogel prepared by the conventional method showed %50 Hase (min) of 33 minutes, thereby confirming that the crosslinked hyaluronic acid hydrogel according to the present invention showed relatively high enzyme resistance.

Experimental example 2-2: ***In vitro* enzyme resistance investigation (2)**

The enzyme resistance test was performed in situ according to the following method using a rheometer.

After pulverizing the crosslinked hyaluronic acid hydrogel prepared by Example 1-1 and the crosslinked hyaluronic acid hydrogel of Comparative examples 2 to 4, respectively, and then finally sterilizing, the sterilized sample 1g was weighed to a 50mL tube, and then hyaluronidase 10uL prepared with 500unit/mL was added and mixed. The sample mixed with the enzyme was loaded on the rheometer and the temperature was set to 37°C, and then the complex viscosity of the sample was measured in real time. Based on the measured result, %50 Hase (min) was calculated and the result was shown in FIG. 1.

As confirmed in FIG. 1, the %50 Hase (min) of Example 1-1 for crosslinked hyaluronic acid hydrogel according to the present invention was about 32 minutes, while Comparative examples 2 to 4 that were crosslinked hyaluronic acid hydrogel prepared by the conventional method showed %50 Hase (min) of 10, 4 and 6 minutes, respectively, thereby confirming that the crosslinked hyaluronic acid hydrogel according to the present invention showed three or more times higher enzyme resistance.

Experimental example 3-1: ***In vitro* radical resistance investigation (1)**

In order to measure resistance to degradation induced by peroxy radicals of the crosslinked hyaluronic acid hydrogel prepared by Example 1-1 and the crosslinked hyaluronic acid hydrogel of Comparative examples 2 to 4, as same as the enzyme resistance experiment, it was measured by the following method using a rheometer in real time.

After weighing the sterilized sample 1g into a 50mL tube, 1 mole hydrogen peroxide ($H_2O_2$) 10uL and 1 mole ascorbic acid 10uL were added and mixed. The mixed sample was loaded on the rheometer and the temperature was set to 37°C, and then the complex viscosity of the sample was measured for 6 hours in real time. From the result, %50 $H_2O_2$ (min) was calculated and shown in FIG. 2. %50 $H_2O_2$ (min) means time it takes for the complex viscosity of crosslinked hyaluronic acid hydrogel to decrease to 50% of its initial physical properties (complex viscosity) due to decomposition by reactive oxygen species (radicals). In other words, it means that the higher the value of %50 $H_2O_2$ (min), the higher the resistance of the crosslinked hyaluronic acid hydrogel to reactive oxygen species (radicals).

As confirmed in FIG. 2, the %50 $H_2O_2$ (min) of Example 1 for crosslinked hyaluronic acid hydrogel according to the present invention was about 32 minutes, while Comparative examples 2 to 4 that were commercially available crosslinked hyaluronic acid hydrogel showed %50 $H_2O_2$ (min) of 5, 7.5 and 18 minutes, respectively, thereby confirming that the crosslinked hyaluronic acid hydrogel of Example 1-1 according to the present invention showed three or more times higher enzyme resistance.

Experimental example 3-2: ***In vitro* radical resistance investigation (2)**

In order to measure resistance to degradation induced by peroxy radicals of the crosslinked hyaluronic acid hydrogel comprising sugar alcohols prepared by Examples 1-2, 2 and 3 and the crosslinked hyaluronic acid hydrogel of Comparative example 1, as same as the enzyme resistance experiment, it was measured by the following method using a rheometer in real time.

After weighing the sterilized sample 1g into a 50mL tube, 1 mole hydrogen peroxide ($H_2O_2$) 10uL and 1 mole ascorbic acid 10uL were added and mixed. The mixed sample was loaded on the rheometer and the temperature was set to 37°C, and then the complex viscosity of the sample was measured for 6 hours in real time. From the result, %50

$H_2O_2$ (min) was calculated and shown in Table 3. %50 $H_2O_2$ (min) means time it takes for the complex viscosity of crosslinked hyaluronic acid hydrogel to decrease to 50% of its initial physical properties (complex viscosity) due to decomposition by reactive oxygen species (radicals). In other words, it means that the higher the value of %50 $H_2O_2$ (min), the higher the resistance of the crosslinked hyaluronic acid hydrogel to reactive oxygen species (radicals).

[Table 3]

| Sample | %50 $H_2O_2$ (Minute) |
|---|---|
| Example 1-2 | 16.5 |
| Example 2 | 18.4 |
| Example 3 | 17.6 |
| Comparative example 1 | 14.6 |

As confirmed in Table 3, the %50 $H_2O_2$ (min) of Examples 1-2, 2 and 3 for crosslinked hyaluronic acid hydrogel comprising sugar alcohol according to the present invention was 16.5 minutes, 18.4 minutes and 17.6 minutes, respectively, while Comparative example 1 that was the crosslinked hyaluronic acid hydrogel prepared by the conventional method showed %50 $H_2O_2$ (min) of 14.6 minutes, thereby confirming that the crosslinked hyaluronic acid hydrogel according to the present invention showed relatively high radical resistance.

Experimental example 4: ***In vitro* heat resistance investigation**

The crosslinked hyaluronic acid hydrogel prepared by Example 1-1 and the crosslinked hyaluronic acid hydrogel of Comparative examples 2 to 4 were stored under severe conditions (55°C±2°C, 75% RH±5% RH) for 4 hours, and the complex viscosity at 0.02Hz was measured using a rheometer, and the residual rate (%) of complex viscosity was calculated and the result was shown in FIG. 3 (unit: cP). The complex viscosity residual rate (%) represents the ratio of the current complex viscosity to the initial complex viscosity, and it means that the larger the value, the better the stability of the physical properties (complex viscosity).

As shown in FIG. 3, in the crosslinked hyaluronic acid hydrogel of Example 1-1 according to the present invention, the residual rate of complex viscosity of about 93% or more remains (is maintained) compared to the crosslinked hyaluronic acid of Comparative examples 1 to 3, while Comparative examples 2 to 4 show a low complex viscosity residual rate, and therefore, it can be confirmed that the crosslinked hyaluronic acid hydrogel according to the present invention shows very excellent stability to heat.

Experimental example 5: ***In vivo* biocompatibility investigation**

In order to confirm the biocompatibility of the crosslinked hyaluronic acid hydrogel prepared by Example 1-1 and the crosslinked hyaluronic acid hydrogel of Comparative examples 5 to 7, a test as follows was performed.

Using male rabbits ((New Zealand White rabbit, Orient Bio), 0.3mL of the sample was administered subcutaneously to 6 mice per group, and after 4 weeks, histopathology was performed. It was experimented according to ISO10993-6 Annex E (Examples of evaluation of local biological effects after implantation). The result of skin reaction was evaluated by the irritation index. The irritation index is an evaluation value of tissue reaction of the substance when a substance is administered to an animal, and means that the higher the value, the lower the biocompatibility, and Non-irritant was evaluated as 0.0~2.9, and Slightly irritant was as 3.0~8.9, and Moderately irritant was as 9.0~15.0, and Severely irritant was as >15.0, and the result was shown in FIG. 4. As shown in FIG. 4, the irritation index of Comparative examples 5 and 6 is about 18 and 8, respectively, whereas the irritation index of Example 1 shows an irritation index value of about 5, and therefore, it exhibits a low or similar level compared to commercially available wrinkle-improving fillers, and thus, it could be confirmed that it exhibited appropriate biocompatibility as a filler to be injected into the body.

**Claims**

**1.** A crosslinked hyaluronic acid hydrogel,

comprising hyaluronic acid or its salt, a crosslinking agent and a polyol,
wherein the hyaluronic acid or its salt is crosslinked with the crosslinking agent and polyol.

2. The crosslinked hyaluronic acid hydrogel according to claim 1, wherein the salt of hyaluronic acid is selected from the group consisting of sodium hyaluronate, potassium hyaluronate, calcium hyaluronate, magnesium hyaluronate, zinc hyaluronate, cobalt hyaluronate and tetrabutylammonium hyaluronate.

3. The crosslinked hyaluronic acid hydrogel according to claim 1, wherein the hyaluronic acid or its salt has an average molecular weight of 1,000,000 to 4,000,000 Da and is crosslinked.

4. The crosslinked hyaluronic acid hydrogel according to claim 1, wherein the crosslinking agent is one or more agents selected from the group consisting of endogenous polyamine, aldehyde, carbodiimide, divinylsulfone, butanediol diglycidyl ether (1,4-butanediol diglycidyl ether: BDDE), ethylene glycol diglycidyl ether (EGDGE), hexanediol diglycidyl ether (1,6-hexanediol diglycidyl ether), propylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, tri-methylpropane polyglycidyl ether, bisepoxypropoxy ethylene (1,2-(bis(2,3-epoxypropoxy)ethylene), pentaerythritol polyglycidyl ether and sorbitol polyglycidyl ether.

5. The crosslinked hyaluronic acid hydrogel according to claim 1, wherein the polyol is a polyol having 2 to 20 carbon atoms.

6. The crosslinked hyaluronic acid hydrogel according to claim 5, wherein the polyol is one or more polyols selected from the group consisting of glycerin, 1,3-propanediol, isoprene glycol, pentylene glycol, hexylene glycol; ethylene glycol, propylene glycol, butylene glycol, diethylene glycol, dipropylene glycol, diglycerol, erythritol, arabitol, adonitol, sorbitol, mannitol, xylitol, dulcitol, glucose, fructose, xylose, trehalose, maltose, saccharose, lactose and methylglucoside phosphate.

7. The crosslinked hyaluronic acid hydrogel according to claim 1, having a modification degree of 0.1 to 100.

8. The crosslinked hyaluronic acid hydrogel according to claim 1, comprising a total hyaluronic acid of 10 mg/g to 35 mg/g based on the total weight of the crosslinked hyaluronic acid hydrogel.

9. A method for preparation of the crosslinked hyaluronic acid hydrogel according to claim 1, comprising

(i) mixing hyaluronic acid or its salt with a polyol; and
(ii) adding a mixed solution of a crosslinking agent and an aqueous alkali solution into the mixture of the hyaluronic acid or its salt and polyol for reaction thereof.

10. The method for preparation according to claim 9, wherein the polyol has a concentration of 5 to 100 mol% based on the hyaluronic acid or its salt.

11. The method for preparation according to claim 9, wherein the crosslinking agent has a concentration of 5 to 100 mol% based on the hyaluronic acid or its salt.

12. The method for preparation according to claim 9, wherein a total reaction concentration is 5 to 30 %(w/w) based on the hyaluronic acid weight.

13. The method for preparation according to claim 9, wherein the aqueous alkali solution has a concentration of 0.1 to 0.5 N.

14. The method for preparation according to claim 9, wherein the aqueous alkali solution is NaOH, KOH, NaHCOs, LiOH or a combination thereof.

15. A filler comprising the crosslinked hyaluronic acid hydrogel according to claim 1.

16. The filler according to claim 15, wherein the filler is for skin injection.

17. The filler according to claim 15, wherein the filler is for wrinkle improvement, soft tissue repair or volume enlargement, or contour correction.

18. The filler according to claim 15, further comprising an anesthetic.

**19.** The filler according to claim 18, wherein the anesthetic is selected from the group consisting of ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dycyclonine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, psuedococaine, pyrrocaine, ropivacaine, salicyl alcohol, tetracaine, tolycaine, trimecaine, zolamine, and salts thereof.

**20.** The filler according to claim 15, further comprising a buffer solution and an isotonic agent.

**21.** A method for preparation of the filler according to claim 15, comprising the following steps:

(a) mixing hyaluronic acid or its salt with a polyol;
(b) adding a mixed solution of a crosslinking agent and an aqueous alkali solution into the mixture of the hyaluronic acid or its salt and polyol and reacting thereof to prepare a crosslinked hyaluronic acid hydrogel;
(c) crude cutting the crosslinked hyaluronic acid hydrogel prepared in the (b);
(d) washing and swelling the crude cut hyaluronic acid hydrogel prepared in the (c) using a buffer solution; and
(e) pulverizing the washed and swelled hyaluronic acid hydrogel in the (d).

**22.** A prefilled syringe filled with the filler of claim 15.

**23.** A method for wrinkle improvement, soft tissue repair or volume enlargement, or contour correction, comprising injecting the filler of claim 15.

【FIG. 1】

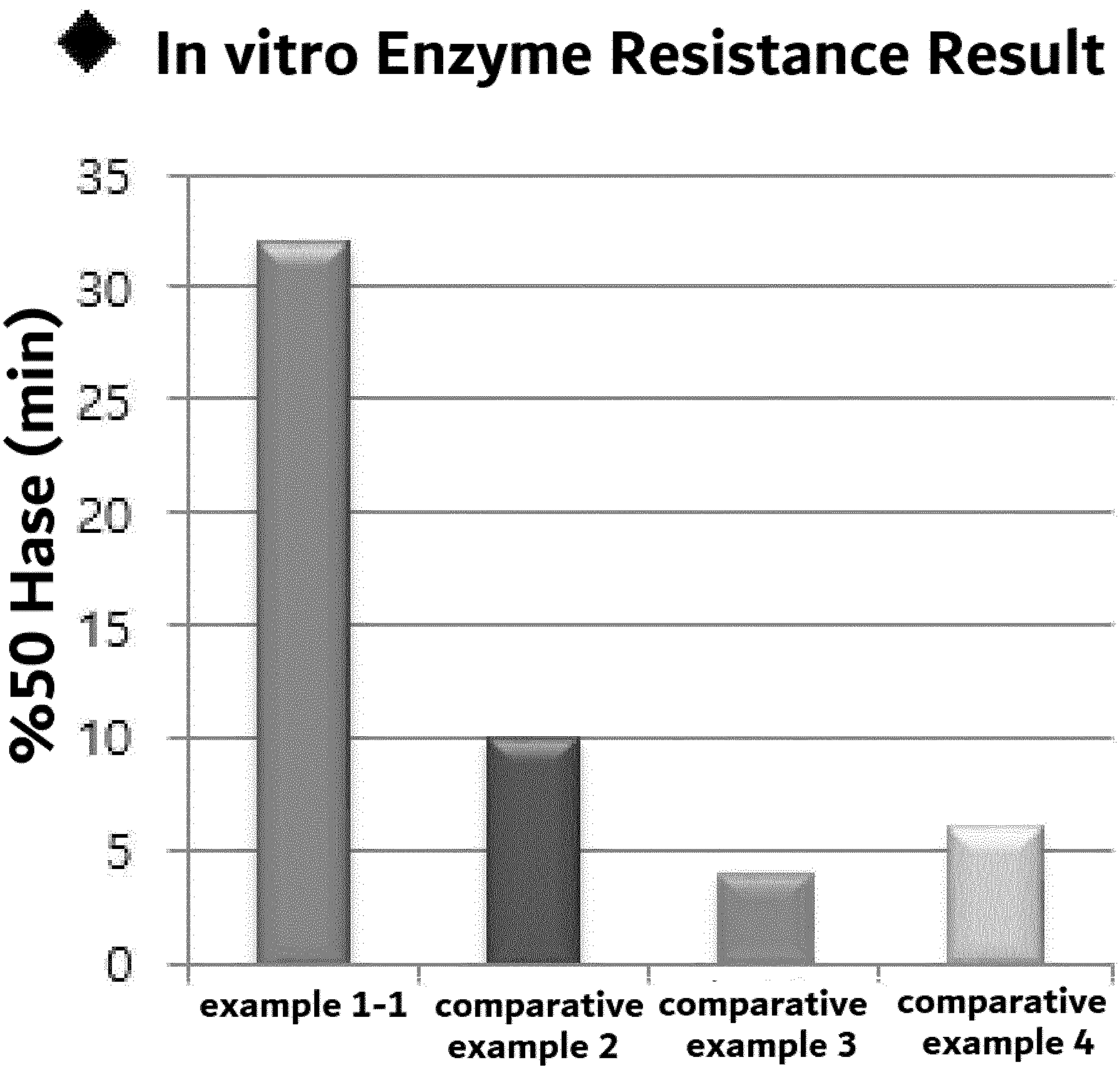
In vitro Enzyme Resistance Result
%50 Hase (min)
35
30
25
20
15
10
5
0
example 1-1
comparative example 2
comparative example 3
comparative example 4

【FIG. 2】

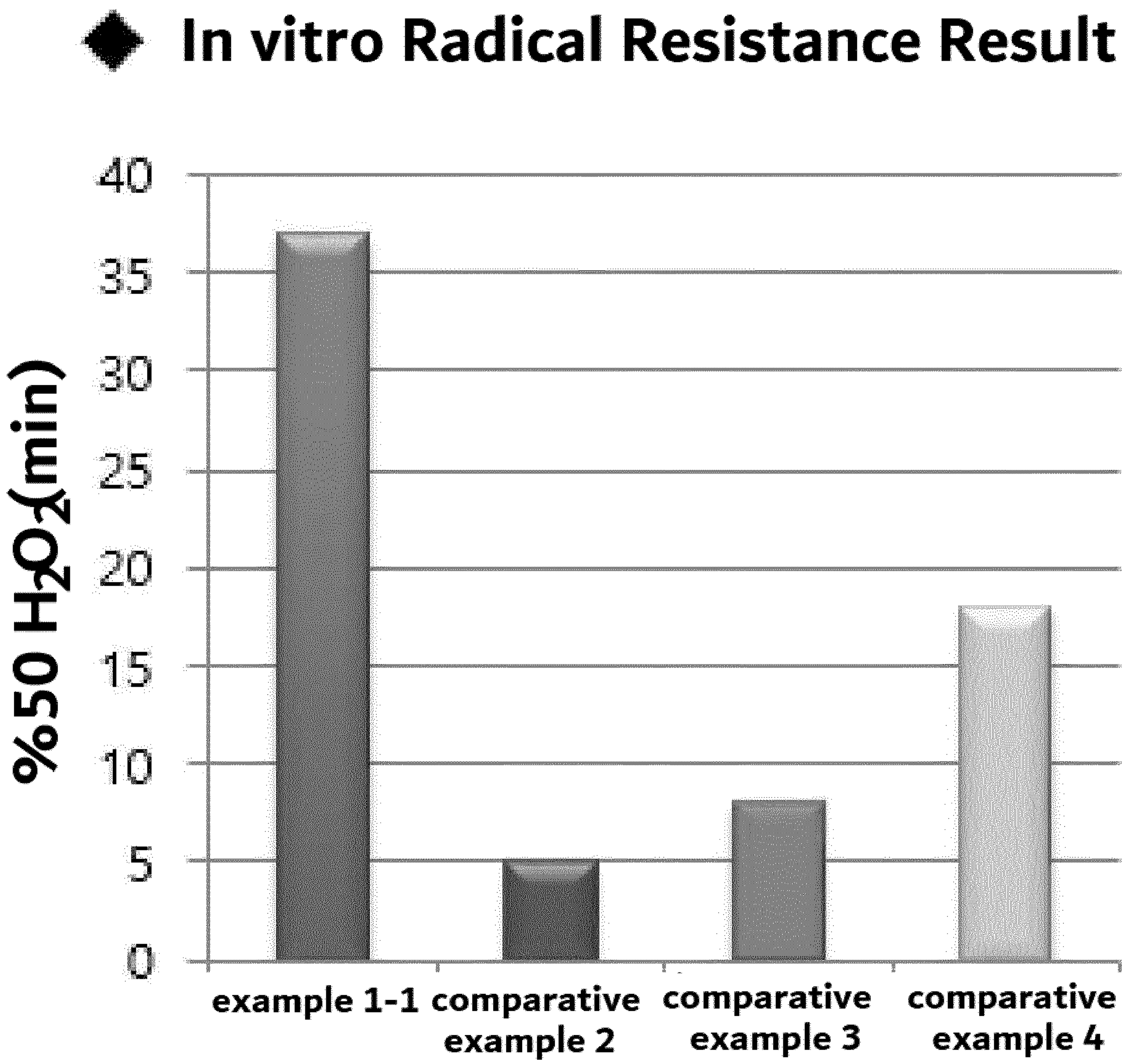
In vitro Radical Resistance Result
%50 H2O2(min)
40
35
30
25
20
15
10
5
0
example 1-1
comparative example 2
comparative example 3
comparative example 4

【FIG. 3】

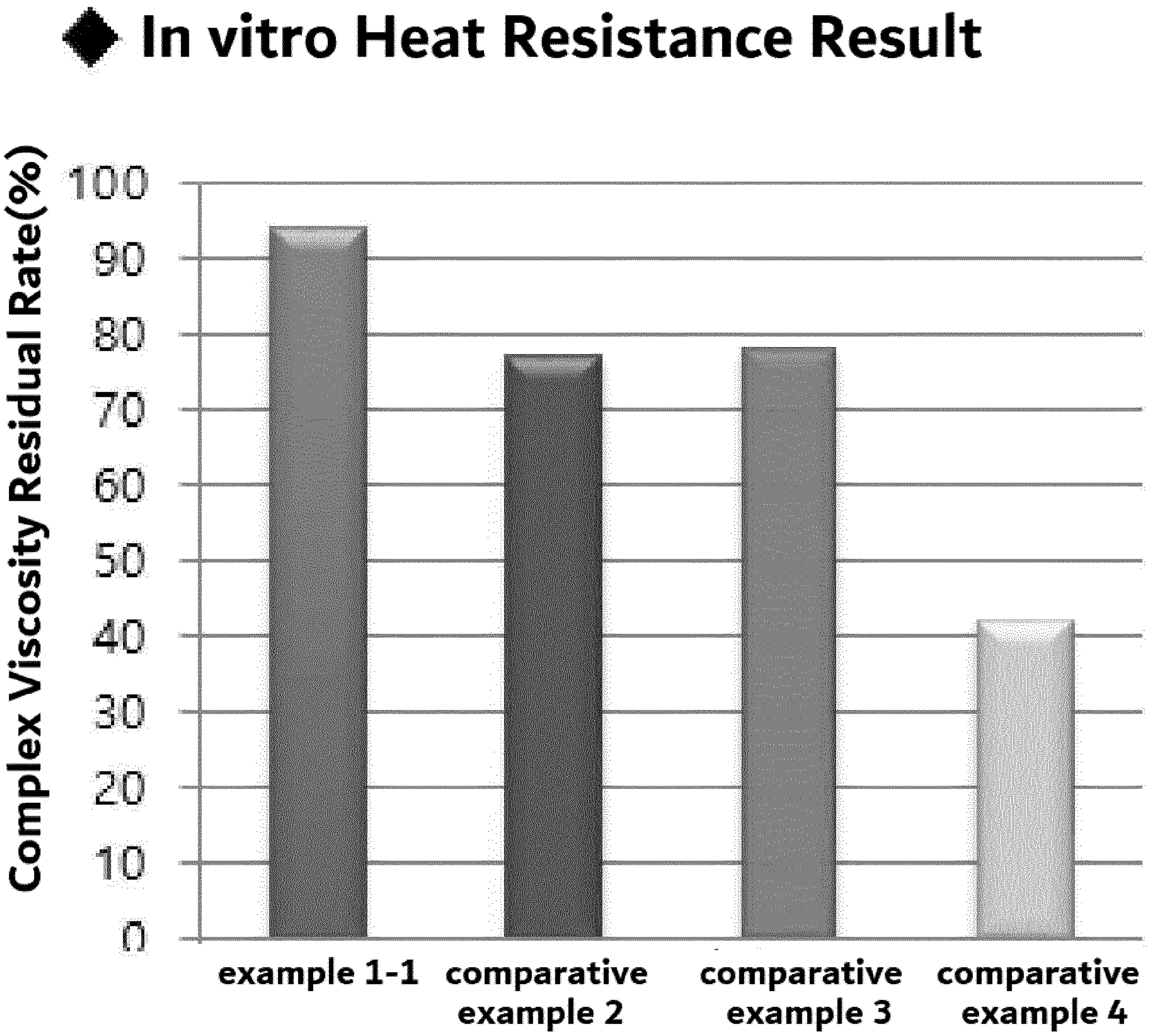

◆ In vitro Heat Resistance Result
Complex Viscosity Residual Rate(%)
100
90
80
70
60
50
40
30
20
10
0
example 1-1
comparative example 2
comparative example 3
comparative example 4

【FIG. 4】

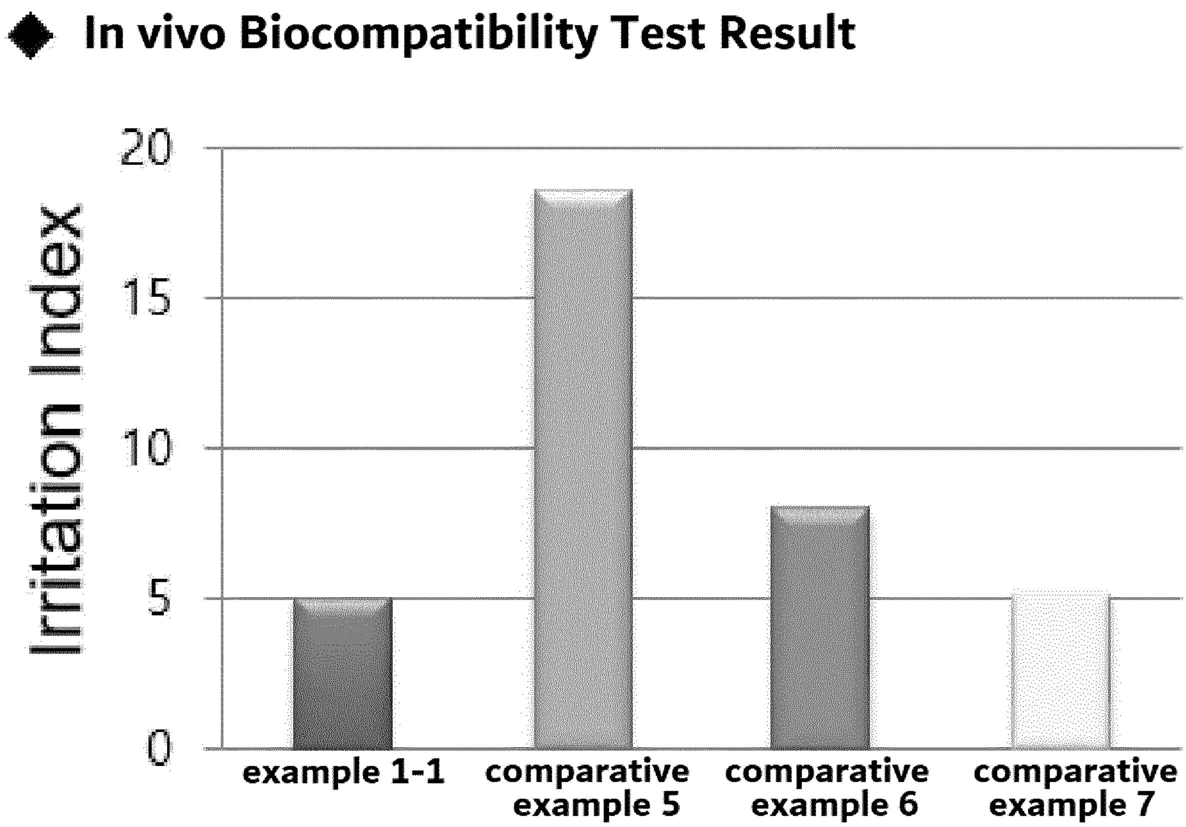
In vivo Biocompatibility Test Result
Irritation Index
20
15
10
5
0
example 1-1
comparative example 5
comparative example 6
comparative example 7

**INTERNATIONAL SEARCH REPORT**

International application No. **PCT/KR2022/000338**

**A. CLASSIFICATION OF SUBJECT MATTER**

**A61L 27/52**(2006.01)i; **A61L 27/20**(2006.01)i; **C08B 37/08**(2006.01)i; **C08J 3/075**(2006.01)i; **A61M 5/28**(2006.01)i

According to International Patent Classification (IPC) or to both national classification and IPC

**B. FIELDS SEARCHED**

Minimum documentation searched (classification system followed by classification symbols)

A61L 27/52(2006.01); A61F 2/00(2006.01); A61K 31/047(2006.01); A61K 31/381(2006.01); A61K 31/728(2006.01); A61K 8/64(2006.01); A61L 27/20(2006.01); A61L 27/54(2006.01); A61L 9/14(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 필러(filler), 히알루론산(hyaluronic acid), 하이드로겔(hydrogel), 가교(cross-linking), 폴리올(polyol)

**C. DOCUMENTS CONSIDERED TO BE RELEVANT**

| Category* | Citation of document, with indication, where appropriate, of the relevant passages | Relevant to claim No. |
|---|---|---|
| A | KIM, E. S. et al. Improving the Rheological Properties of Cross-linked Hyaluronic Acid Gels by Mixing in Mannitol as a Radical Scavenger. Asian J. Beauty Cosmetol. 2019, vol. 17, no. 1, pp. 57-67. See abstract; and pages 58-59. | 1-23 |
| A | WO 2020-009555 A1 (LG CHEM, LTD.) 09 January 2020 (2020-01-09) See claim 14. | 1-23 |
| A | KR 10-2127060 B1 (ALLERGAN, INC. et al.) 26 June 2020 (2020-06-26) See entire document. | 1-23 |
| A | KR 10-2018-0004256 A (LABORATOIRES VIVACY) 10 January 2018 (2018-01-10) See entire document. | 1-23 |
| A | KR 10-1514831 B1 (ANTEIS S.A.) 24 April 2015 (2015-04-24) See entire document. | 1-23 |

☑ Further documents are listed in the continuation of Box C.　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 April 2022** | **14 April 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2022/000338**

**C. DOCUMENTS CONSIDERED TO BE RELEVANT**

| Category* | Citation of document, with indication, where appropriate, of the relevant passages | Relevant to claim No. |
|---|---|---|
| A | US 8574629 B2 (GAVARD MOLLIARD, S.) 05 November 2013 (2013-11-05)<br>See entire document. | 1-23 |

**INTERNATIONAL SEARCH REPORT**
**Information on patent family members**

International application No.
**PCT/KR2022/000338**

| Patent document cited in search report | Publication date (day/month/year) | Patent family member(s) | Publication date (day/month/year) |
|---|---|---|---|
| WO 2020-009555 A1 | 09 January 2020 | AU 2019-299812 A1 | 25 February 2021 |
| | | AU 2019-299812 A2 | 18 March 2021 |
| | | BR 112021000188 A2 | 01 June 2021 |
| | | CA 3105709 A1 | 09 January 2020 |
| | | CN 112423798 A | 26 February 2021 |
| | | EP 3804769 A1 | 14 April 2021 |
| | | KR 10-2020-0005505 A | 15 January 2020 |
| | | TW 202005656 A | 01 February 2020 |
| | | TW I708607 B | 01 November 2020 |
| | | US 2021-0268143 A1 | 02 September 2021 |
| KR 10-2127060 B1 | 26 June 2020 | AU 2011-206389 A1 | 09 August 2012 |
| | | AU 2011-206389 B2 | 02 July 2015 |
| | | CA 2905371 A1 | 21 July 2011 |
| | | CA 2905371 C | 16 August 2016 |
| | | EP 3511028 A1 | 17 July 2019 |
| | | EP 3511028 B1 | 07 April 2021 |
| | | EP 3682911 A1 | 22 July 2020 |
| | | EP 3862029 A1 | 11 August 2021 |
| | | JP 2013-517263 A | 16 May 2013 |
| | | JP 2014-513988 A | 19 June 2014 |
| | | JP 2015-205921 A | 19 November 2015 |
| | | JP 2016-166254 A | 15 September 2016 |
| | | JP 2017-210491 A | 30 November 2017 |
| | | JP 2019-131586 A | 08 August 2019 |
| | | JP 5788412 B2 | 30 September 2015 |
| | | JP 5960894 B2 | 02 August 2016 |
| | | JP 6152345 B2 | 21 June 2017 |
| | | JP 6208293 B2 | 04 October 2017 |
| | | JP 6509979 B2 | 08 May 2019 |
| | | KR 10-1768733 B1 | 30 August 2017 |
| | | KR 10-1830591 B1 | 20 February 2018 |
| | | KR 10-2012-0125293 A | 14 November 2012 |
| | | KR 10-2014-0033337 A | 18 March 2014 |
| | | KR 10-2017-0094463 A | 17 August 2017 |
| | | KR 10-2018-0101613 A | 12 September 2018 |
| | | KR 10-2020-0078672 A | 01 July 2020 |
| | | KR 10-2111170 B1 | 15 May 2020 |
| | | US 10220113 B2 | 05 March 2019 |
| | | US 10449268 B2 | 22 October 2019 |
| | | US 10806821 B2 | 20 October 2020 |
| | | US 2011-0171286 A1 | 14 July 2011 |
| | | US 2011-0171310 A1 | 14 July 2011 |
| | | US 2011-0171311 A1 | 14 July 2011 |
| | | US 2011-0172180 A1 | 14 July 2011 |
| | | US 2012-0208890 A1 | 16 August 2012 |
| | | US 2012-0225842 A1 | 06 September 2012 |
| | | US 2012-0232030 A1 | 13 September 2012 |
| | | US 2013-0072453 A1 | 21 March 2013 |
| | | US 2015-0038456 A1 | 05 February 2015 |
| | | US 2015-0306280 A1 | 29 October 2015 |

Form PCT/ISA/210 (patent family annex) (July 2019)

**INTERNATIONAL SEARCH REPORT**
**Information on patent family members**

International application No.
**PCT/KR2022/000338**

| Patent document cited in search report | Publication date (day/month/year) | Patent family member(s) | Publication date (day/month/year) |
|---|---|---|---|
| | | US 2015-0314040 A1 | 05 November 2015 |
| | | US 2016-0220729 A1 | 04 August 2016 |
| | | US 2017-0216488 A1 | 03 August 2017 |
| | | US 2018-0078674 A1 | 22 March 2018 |
| | | US 2019-0192732 A1 | 27 June 2019 |
| | | US 2021-0077658 A1 | 18 March 2021 |
| | | US 8946192 B2 | 03 February 2015 |
| | | US 9114188 B2 | 25 August 2015 |
| | | US 9333160 B2 | 10 May 2016 |
| | | US 9655991 B2 | 23 May 2017 |
| | | US 9855367 B2 | 02 January 2018 |
| | | WO 2011-086458 A1 | 21 July 2011 |
| | | WO 2012-097272 A1 | 19 July 2012 |
| | | WO 2012-097272 A8 | 20 September 2012 |
| KR 10-2018-0004256 A | 10 January 2018 | CA 2985695 A1 | 17 November 2016 |
| | | CN 107735084 A | 23 February 2018 |
| | | EP 3294355 A1 | 21 March 2018 |
| | | FR 3036035 A1 | 18 November 2016 |
| | | FR 3036035 B1 | 18 November 2016 |
| | | HK 1249436 A1 | 02 November 2018 |
| | | MA 42470 A | 28 April 2021 |
| | | WO 2016-180904 A1 | 17 November 2016 |
| KR 10-1514831 B1 | 24 April 2015 | CN 102196805 A | 21 September 2011 |
| | | CN 102196805 B | 16 October 2013 |
| | | EP 2349203 A2 | 03 August 2011 |
| | | EP 2349203 B1 | 30 October 2013 |
| | | EP 2349203 B2 | 26 February 2020 |
| | | EP 2676658 A1 | 25 December 2013 |
| | | EP 2676658 B1 | 16 September 2015 |
| | | ES 2443296 T3 | 18 February 2014 |
| | | ES 2443296 T5 | 29 October 2020 |
| | | ES 2556475 T3 | 18 January 2016 |
| | | FR 2938187 A1 | 14 May 2010 |
| | | FR 2938187 B1 | 14 May 2010 |
| | | JP 2012-508217 A | 05 April 2012 |
| | | JP 5626661 B2 | 19 November 2014 |
| | | KR 10-2011-0084510 A | 25 July 2011 |
| | | RU 2011121426 A | 10 December 2012 |
| | | RU 2493815 C2 | 27 September 2013 |
| | | US 2011-0201571 A1 | 18 August 2011 |
| | | US 8455465 B2 | 04 June 2013 |
| | | WO 2010-052430 A2 | 14 May 2010 |
| | | WO 2010-052430 A3 | 07 October 2010 |
| US 8574629 B2 | 05 November 2013 | JP 2010-036032 A | 18 February 2010 |
| | | JP 5574083 B2 | 20 August 2014 |
| | | US 2010-0028435 A1 | 04 February 2010 |

Form PCT/ISA/210 (patent family annex) (July 2019)